# EUROPEAN PATENT APPLICATION

(11) **EP 1 975 166 A1**
(43) Date of publication of application: **01.10.2008**
(21) Application number: 07090057.6
(22) Date of filing: 30.03.2007
(51) Int. Cl.: C07D 401/12

(54) **Synthesis of anthranilamides**

(71) Applicant: Bayer Schering Pharma AG, 13353 Berlin (DE)
(72) Inventor: Schmees, Norbert, 13469 Berlin (DE); Petrov, Orlin, 14195 Berlin (DE)

(57) **Abstract**

The present invention relates to a novel method of preparing anthranilamides of formula I : 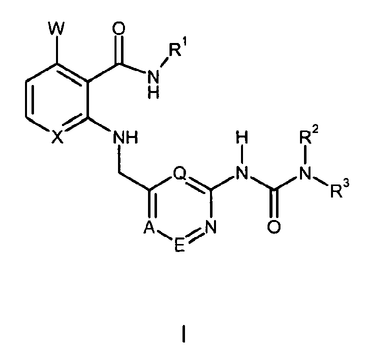 in which R¹ , R² , R³ , A, E, Q, X, and W are defined in the claims, to a novel intermediate compound, and to the use of said novel intermediate compound for the preparation of said anthranilamides of formula I.

## Description

### FIELD OF THE INVENTION

The present invention relates to a novel method of preparing anthranilamides, to a novel intermediate compound, and to the use of said novel intermediate compound for the preparation of said anthranilamides.

### BACKGROUND TO THE INVENTION

Certain anthranilamides, particularly anthranilamide pyridinureas, are known to be VEGF receptor kinase inhibitors, which are useful as pharmaceutical agents for preventing or treating diseases that are triggered by persistent angiogenesis. Diseases which are associated with persistent angiogenesis are, for example, diseases such as tumor- or metastases-growth ; psoriasis ; arthritis, such as rheumatoid arthritis, hemangioma, endometriosis, angiofibroma ; eye diseases, such as diabetic retinopathy, neovascular glaucoma ; renal diseases, such as glomerulonephritis, diabetic nephropathy, malignant nephrosclerosis, thrombotic microangiopathic syndrome, transplant rejections and glomerulopathy ; fibrotic diseases, such as cirrhosis of the liver, mesangial cell proliferative diseases and arteriosclerosis.

International Patent Application publications WO 2006/048248, WO 2006/048249, and WO 2006/048251, for example, which are hereby incorporated in their entirety by reference, describe such anthranilamides and their synthesis.

Said anthranilamides can be synthesised in accordance with the above-mentioned publications, which describe syntheses which are long multi-step syntheses, which are based on palladium-catalysed carbon-nitrogen couplings as a last synthetic step, and which involve the use of expensive reagents, e.g. palladium containing reagents, and have the drawback that elimination of palladium in the last step from the final product is very difficult, making the final product less suitable as a pharmaceutical agent.

In particular, said anthranilamides can be synthesised via the following general synthetic Scheme 1, *infra :*

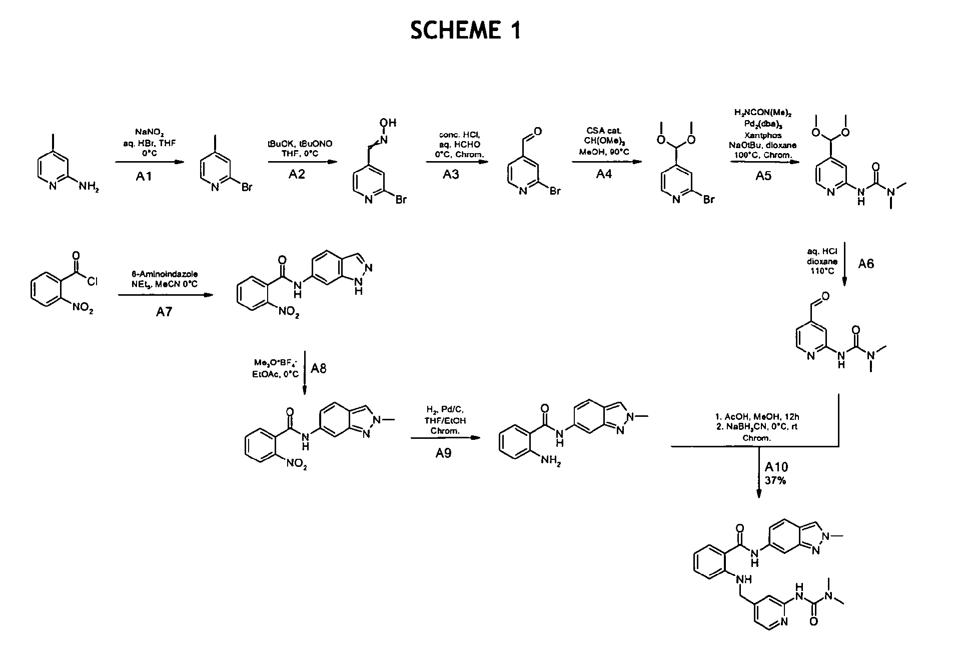

The synthesis as outlined in Scheme 1, *supra*, suffers from the numerous technical drawbacks in that it involves a long 10-step synthesis which necessitates four chromatographic purification steps, particularly for the purification of amine A9, of acetal A5, and of the final anthranilamide A10. Furthermore, the synthesis of Scheme 1 provides only a 20% overall yield, the last step A10 only providing 37% yield. Moreover, the synthesis of Scheme 1 involves very important safety concerns which are well known to the person skilled in the art, particularly with regard to the potentially hazardous use of 2-nitrobenzoyl chloride in the first step, and to the use of tert-butyl nitrite in the preparation of the intermediate oxime as shown in Scheme 1. Finally, the synthesis according to Scheme 1 involves the use of some very expensive reagents, for example trimethyloxonium tetrafluoroborate ("Meerwein's salt") as methylating agent in step A8, Xanthphos and Pd₂(dba)₃ in step A5.

It was therefore highly desirable to have a method of synthesising anthanilamides at one's disposal which alleviates the important technical problems set forth above, and which can be used on an industrial scale.

It has been very surprisingly discovered, and this provides the basis of the present invention, that anthranilamides can be synthesised in accordance with Scheme 2, *infra :*

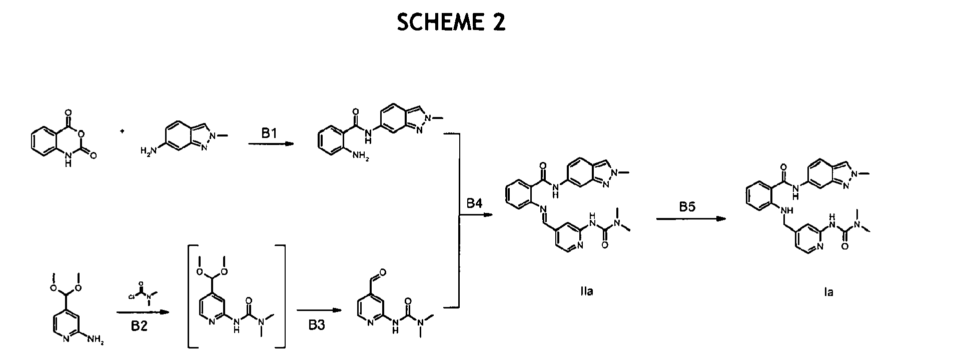

Scheme 2 represents an illustration of the various steps used in the method according to the invention.

In step B1, which can be carried out according to methods which are known to the person skilled in the art, the anilide derivative is generated in accordance with Example 1 of the invention, and is purified by crystallisation. Step B2 builds up the urea part in high yield, step B2 can be carried out according to methods which are known to the person skilled in the art, particularly J. Med. Chem., 1983, 538 or US 4,203,988. The intermediate is optionally isolated and can be used as a solution for the following acid catalysed cleavage of the acetal to provide the aldehyde product of step B3, step B3 being carried out according to methods which are known to the person skilled in the art, particularly by hydrolysis with 2N HCl, optionally with heating, particularly under reflux at 90 degree centrigrade, followed by neutralisation with a base, for example potassium phosphate, extraction, followed by optional purification by crystallisation or chromatography. The imine of formula IIa can be formed out of the two intermediates from steps B1 and B3, i.e. the above-mentioned anilide and aldehyde, respectively, and can be isolated by crystallisation. This imine IIa is the precursor for the generation of anthranilamides of formula I by reduction of the C=N double bond.

The advantages of the method according to the invention, as in Scheme 2, *supra,* are, *inter alia,* that the problems associated with safety of reagents is overcome, process efficiency is increased, overall yield is greatly improved from 20 to 63%, all chromatographic purification steps are eliminated, and, importantly, decreasing the time of manufacture of drug substance.

More particularly, in the last step of the synthesis according to the present invention, a 79 % yield of the final anthranilamide I was recorded, together with a purity of 98.5%, this represents an unexpected technical improvement over prior art methods. More surprisingly, it was discovered that the intermediate imine of formula II was highly crystalline : it is extremely insoluble. The extreme insolubility of the imine II provides a most unexpected technical advantage, in that it can be isolated in pure form by simple filtration of the precipitate, (or indeed by crystallisation from a range of solvents), thereby very much improving the efficiency of the process, particularly on an industrial scale. All in all, the synthetic method according to the invention proved to be very advantageous with regard to economical production of anthranilamides. The prior art technical problems set forth *supra,* particularly in relation to the synthesis of scheme 1, *supra*, are surprisingly overcome.

Hence, in accordance with a first aspect, the present invention relates to a method of preparing an anthranilamide of formula I : 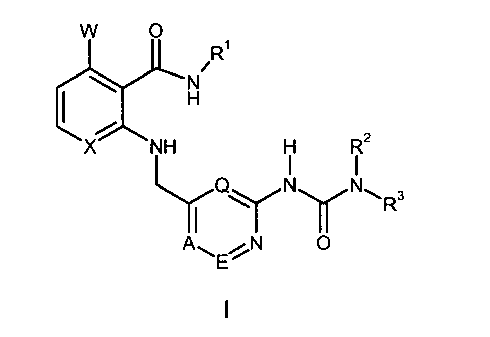 in which :
- X: is CH or N ;
- W: is hydrogen or fluorine ;
- A, E: and Q, independently of one another, are CH or N, whereby only a maximum of two nitrogen atoms are contained in the ring ;
- R¹: is aryl or heteroaryl, which may be optionally substituted in one or more places in the same way or differently with halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴,-COR⁶, -CO₂R⁶ or -NR⁷R⁸, whereby C₁-C₁₂-alkyl may be substituted with -OR⁵ or-NR⁷R⁸,
- R² and R³,: independently of one another, are C₁-C₁₂ alkyl optionally substituted with -OR⁵ ;
- R⁴: is C₁-C₁₂-alkyl, C₃-C₈-cycloalkyl, aryl or heteroaryl ;
- R⁵: is hydrogen, C₁-C₁₂-alkyl, C₃-C₈-cycloalkyl or halo-C₁-C₆-alkyl ;
- R⁶: is hydrogen, C₁-C₁₂-alkyl, C₃-C₈-cycloalkyl, halo-C₁-C₆-alkyl, aryl, or-NR⁷R⁸ ;
- R⁷ and R⁸: independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, preferably a 4-7 membered cycloalkyl ring, more preferably a 5 or 6 membered cycloalkyl ring, which may optionally contain further heteroatoms, and may be optionally substituted in one or more places in the same way or differently with halogen, cyano, C₁-C₁₂-alkyl, C₁-C₁₂-alkoxy, halo-C₁-C₆-alkyl, =O, -OR⁵ , COR⁶, -SR⁴, -SOR⁴ or -SO₂R⁶ ; and
as well as isomers, diastereoisomers, enantiomers, tautomers thereof,
characterised in that an imine of formula II : 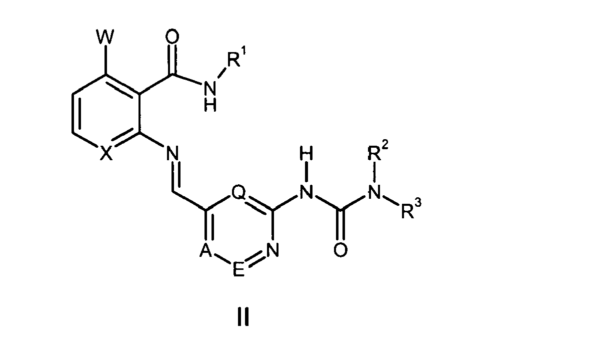 in which R¹ , R² , R³ , A, E, Q, X, and W are defined as for formula I,
is allowed to react with a reducing agent, optionally in the presence of a solvent, thereby providing an anthranilamide of formula I.

According to a preferred embodiment of the first aspect, the invention provides a method as described *supra,* wherein in said compound of formula I,
- X: is CH or N ;
- W: is hydrogen or fluorine ;
- A, E and Q,: independently of one another, are CH or N, whereby only a maximum of two nitrogen atoms are contained in the ring ;
- R¹: is aryl or heteroaryl, which may be optionally substituted in one or more places in the same way or differently with halogen, hydroxy, C₁-C₁₂-alkyl, C₁-C₁₂-alkoxy, halo-C₁-C₆-alkyl, -SO₂R⁶, -OR⁵, COR⁶, -CO₂R⁶ or -NR⁷R⁸, whereby C₁-C₁₂-alkyl may be substituted with -OR⁵ or -NR⁷R⁸,
- R² and R³,: independently of one another, are C₁-C₁₂ alkyl optionally substituted with -OR⁵ ;
- R⁴: is C₁-C₁₂-alkyl, C₃-C₈-cycloalkyl, aryl or heteroaryl ;
- R⁵: is hydrogen, C₁-C₁₂-alkyl, C₃-C₈-cycloalkyl or halo-C₁-C₆-alkyl ;
- R⁶: is aryl, or -NR⁷R⁸ ;
- R⁷ and R⁸: independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, preferably a 4-7 membered cycloalkyl ring, more preferably a 5 or 6 membered cycloalkyl ring, which may optionally contain further heteroatoms, and may be optionally substituted in one or more places in the same way or differently with halogen, C₁-C₁₂-alkyl, C₁-C₁₂-alkoxy, halo-C₁-C₆-alkyl, -OR⁵, COR⁶, -SR⁴, or -SO₂R⁶ ; and
as well as isomers, diastereoisomers, enantiomers, tautomers thereof.

According to a more preferred embodiment of the first aspect, the invention provides a method as described *supra*, wherein in said compound of formula I,
- X: is CH ;
- W: is hydrogen ;
- A, E and Q,: independently of one another, are CH or N, whereby only a maximum of two nitrogen atoms are contained in the ring ;
- R¹: is aryl or heteroaryl, which may be optionally substituted in one or more places in the same way or differently with C₁-C₁₂-alkyl, C₁-C₁₂-alkoxy,-SO₂R⁶, -OR⁵, COR⁶, or -NR⁷R⁸, whereby C₁-C₁₂-alkyl may be substituted with-OR⁵ -NR⁷R⁸,
- R² and R³,: independently of one another, are C₁-C₁₂ alkyl optionally substituted with -OR⁵ ;
- R⁵: is C₁-C₁₂-alkyl, or C₃-C₈-cycloalkyl ;
- R⁶: is aryl, or -NR⁷R⁸ ;
- R⁷ and R⁸: independently of one another, are hydrogen, -COR⁶, aryl, C₃-C₈-cycloalkyl, C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, preferably a 4-7 membered cycloalkyl ring, more preferably a 5 or 6 membered cycloalkyl ring, which may optionally contain further heteroatoms, and may be optionally substituted in one or more places in the same way or differently with halogen, C₁-C₁₂-alkyl, C₁-C₁₂-alkoxy, -OR⁵ , COR⁶, or -SO₂R⁶ ; and
as well as isomers, diastereoisomers, enantiomers, tautomers thereof.

According to a more preferred embodiment of the first aspect, the invention provides a method as described *supra,* wherein said compound of formula I is selected from the group consisting of :
2-{[2-(3,3-dimethyl-ureido)-pyridin-4-ylmethyl]-amino}-N-(2-methyl-2H-indazol-6-yl)-benzamide
2-{[2-(3,3-diethyl-ureido)-pyridin-4-ylmethyl]-amino}-N-(2-methyl-2H-indazol-6-yl)-benzamide
2-({2-[3-(2-hydroxy-ethyl)-3-methyl-ureido]-pyridin-4-ylmethyl}-amino)-N-(2-methyl-2H-indazol-6-yl)-benzamide
2-({2-[3-(2-methoxy-ethyl)-3-methyl-ureido]-pyridin-4-ylmethyl}-amino)-N-(2-methyl-2H-indazol-6-yl)-benzamide
2-{[2-(3-ethyl-3-methyl-ureido)-pyridin-4-ylmethyl]-amino}-N-(2-methyl-2H-indazol-6-yl)-benzamide
2-{[2-(3,3-dimethyl-ureido)-pyridin-4-ylmethyl]-amino}-N-(4-fluoro-2-methyl-2H-indazol-6-yl)-benzamide
2-{[2-(3,3-dimethyl-ureido)-pyridin-4-ylmethyl]-amino}-N-(7-methoxy-isoquinolin-3-yl)-benzamide
6-(2-{[2-(3,3-dimethyl-ureido)-pyridin-4-ylmethyl]-amino}-benzoylamino)-2-methyl-2H-indazole-3-carboxylic acid methyl ester
2-{[2-(3,3-dimethyl-ureido)-pyridin-4-ylmethyl]-amino}-N-(2-methyl-2H-benzotriazol-5-yl)-benzamide
2-{[2-(3,3-dimethyl-ureido)-pyridin-4-ylmethyl]-amino}-N-(1-methyl-2-oxo-1,2-dihydro-quinolin-6-yl)-benzamide
2-{[2-(3,3-dimethyl-ureido)-pyridin-4-ylmethyl]-amino}-N-(2-methyl-2H-indazol-7-yl)-benzamide
2-{[2-(3,3-dimethyl-ureido)-pyridin-4-ylmethyl]-amino}-N-(1-methyl-3a,7a-dihydro-1H-indazol-4-yl)-benzamide
2-{[2-(3,3-dimethyl-ureido)-pyridin-4-ylmethyl]-amino}-N-(5-fluoro-2-methyl-2H-indazol-4-yl)-benzamide
2-{[2-(3,3-dimethyl-ureido)-pyridin-4-ylmethyl]-amino}-N-(6-fluoro-2-methyl-2H-indazol-7-yl)-benzamide
6-(2-{[2-(3,3-dimethyl-ureido)-pyridin-4-ylmethyl]-amino}-benzoylamino)-1-methyl-1H-indazole-3-carboxylic acid methyl ester
2-{[2-(3,3-dimethyl-ureido)-pyridin-4-ylmethyl]-amino}-N-(3-hydroxymethyl-1-methyl-1H-indazol-6-yl)-benzamide
N-(3,6-difluoro-quinolin-2-yl)-2-{[2-(3,3-dimethyl-ureido)-pyridin-4-ylmethyl]-amino}-benzamide
2-{[2-(3,3-dimethyl-ureido)-pyridin-4-ylmethyl]-amino}-N-(3-sulfamoyl-phenyl)-benzamide
N-(2,3-dimethyl-2H-indazol-6-yl)-2-{[2-(3,3-dimethyl-ureido)-pyridin-4-ylmethyl]-amino}-benzamide
2-{[2-(3,3-dimethyl-ureido)-pyridin-4-ylmethyl]-amino}-N-(3-methoxymethyl-2-methyl-2H-indazol-6-yl)-benzamide
2-{[2-(3,3-dimethyl-ureido)-pyridin-4-ylmethyl]-amino}-N-(3-methoxymethyl-1-methyl-1H-indazol-6-yl)-benzamide
6-(2-{[2-(3,3-dimethyl-ureido)-pyridin-4-ylmethyl]-amino}-benzoylamino)-1-methyl-1H-indazole-3-carboxylic acid methylamide
2-{[2-(3,3-dimethyl-ureido)-pyridin-4-ylmethyl]-amino}-N-(6-fluoro-1-methyl-1 H-indazol-5-yl)-benzamide
2-{[2-(3, 3-dimethyl-ureido)-pyridin-4-ylmethyl]-amino}-N-(6-fluoro-2-methyl-2H-indazol-5-yl)-benzamide
2-{[2-(3,3-dimethyl-ureido)-pyridin-4-ylmethyl]-amino}-N-(5-fluoro-1-methyl-1 H-indazol-4-yl)-benzamide
2-{[2-(3,3-dimethyl-ureido)-pyridin-4-ylmethyl]-amino}-N-quinolin-3-yl-benzamide
2-{[2-(3,3-dimethyl-ureido)-pyridin-4-ylmethyl]-amino}-N-(3-fluoro-6-methoxy-quinolin-2-yl)-benzamide
2-{[2-(3,3-dimethyl-ureido)-pyridin-4-ylmethyl]-amino}-N-(3-methyl-3H-benzoimidazol-5-yl)-benzamide
2-{[2-(3,3-dimethyl-ureido)-pyridin-4-ylmethyl]-amino}-N-(1-methyl-1H-benzoimidazol-5-yl)-benzamide
2-{[2-(3,3-Dimethyl-ureido)-pyridin-4-ylmethyl]-amino}-N-(3-methanesulfonylphenyl)-benzamide
2-{[2-(3,3-dimethyl-ureido)-pyridin-4-ylmethyl]-amino}-6-fluoro-N-(2-methyl-2H-indazol-6-yl)-benzamide, and
as well as isomers, diastereoisomers, enantiomers, tautomers and salts thereof.

In accordance with a preferred embodiment of the first aspect, the present invention relates to a method of preparing an anthranilamide of formula I, wherein said method, said reducing agent is a hydride ion donor, which is used optionally in the presence of an acid, or a base. More preferably, said hydride donor is selected from the group consisting of complex hydrides like sodium borohydride, sodium cyanoborohydride, sodium triacetoxyborohydride, lithium borohydride, an aluminium hydride such as super hydride, lithium dimethoxyaluminiumhydride, lithium aluminium hydride, an alkylsilane, particularly a trialkylsilane, such as triethylsilane, for example.

In accordance with a variant of the method of the invention, said acid is a mineral acid, or an organic acid, such as a carboxylic acid, such as trifluoroacetic acid, for example.

In accordance with another variant of the method of the present invention, said solvent is selected from the group consisting of water, an alcohol, an ether, a carboxylic ester, and an optionally halogenated hydrocarbon, particularly a chlorinated hydrocarbon, such as dichloromethane, for example.

In accordance with an embodiment of the first aspect, the invention relates to a method as described *supra,* wherein the molar ratio of said imine of formula II/said reducing agent is between 1 : 0.5 and 1:20, preferably between 1: 0.5 and 1:5, particularly between 1:1 and 1:2.

In accordance with an embodiment of the first aspect, the invention relates to a method as described *supra,* wherein the molar ratio of said imine of formula II/said acid is between 1:0.1 and 1:30, particularly between 8 : 1 and 30 :1.

In accordance with an embodiment of the first aspect, the invention relates to a method as described *supra,* which is carried out at a temperature of between the freezing point of the reaction mixture and the reflux temperature of the reaction mixture, preferably between 15°C and 110°C, particularly between room temperature amd 55°C.

In accordance with an embodiment of the first aspect, the invention relates to a method as described *supra,* wherein said imine of formula II is prepared by allowing an amine of formula III : 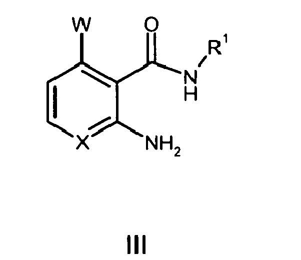 in which R¹ , X, and W are defined as for formula I *supra,*
to react, optionally in the presence of a solvent, with an aldehyde of formula IV : 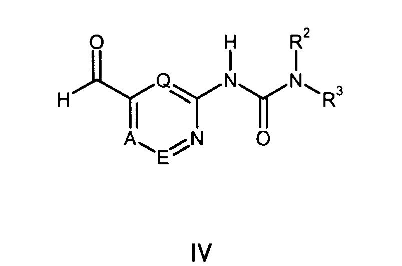 in which R² , R³ , A, E, and Q are defined as for formula I *supra,*
thereby providing an imine of formula II.

In accordance with an embodiment, the reaction of a compound of formula III with a compound of formula IV, *supra*, is carried out under acidic or basic conditions. In an embodiment of the reaction of a compound of formula III with a compound of formula IV, *supra,* said acidic conditions are provided by an acid such as a carboxylic acid, such as acetic acid, for example.

In an embodiment of the reaction of a compound of formula III with a compound of formula IV, *supra,* said solvent is selected from the group consisting of an alcohol, such as methanol, for example, an ether, a carboxylic ester, and an optionally halogenated hydrocarbon, particularly an alcohol, for example methanol and / or ethanol.

In an embodiment of the reaction of a compound of formula III with a compound of formula IV, *supra,* the molar ratio of said amine of formula III/said aldehyde of formula IV is between 1:0.1 and 0.1:1, particularly between 1:0.7 and 0.7:1.

In an embodiment of the reaction of a compound of formula III with a compound of formula IV, *supra,* said acid is present in any amount above 0 moles, preferably at a molar ratio of said amine of formula III/said acid of 1:0.0001 and 1:200, more preferably 1: 0.01 and 1:10, particularly around 1/around 0.1.

In an embodiment of the reaction of a compound of formula III with a compound of formula IV, *supra*, the reaction is carried out at a temperature of between the freezing point of the reaction mixture and the reflux temperature of the reaction mixture, preferably between 0°C and 100°C and particularly between 0°C and 20°C.

According to a second aspect, the present invention relates to an imine of formula II : 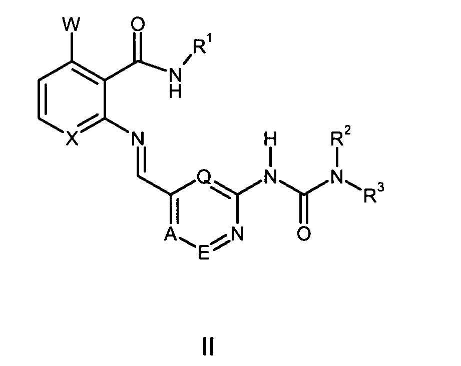 in which R¹ , R² , R³ , A, E, Q, X, and W are defined *supra* as for formula I.

According to a third aspect, the present invention relates to the use of an imine of formula II, *supra,* for the preparation of an anthranilamide of formula I as defined *supra.*

### Definitions of terms used :

As used herein, the term "alkyl" is defined in each case as a substituted or unsubstituted straight-chain or branched alkyl group, such as, for example, methyl, ethyl, propyl, isopropyl, butyl, isobutyl, tert-butyl, pentyl, isopentyl or hexyl, heptyl, octyl, nonyl, decyl, undecyl, or dodecyl.

As used herein, the term "alkoxy" is defined in each case as a straight-chain or branched alkoxy group, such as, for example, methyloxy, ethyloxy, propyloxy, isopropyloxy, butyloxy, isobutyloxy, tert-butyloxy, pentyloxy, isopentyloxy, hexyloxy, heptyloxy, octyloxy, nonyloxy, decyloxy, undecyloxy or dodecyloxy.

As used herein, the term "cycloalkyl" is defined as a monocyclic alkyl ring, such as cyclopropyl, cyclobutyl, cyclopentyl, cyclohexyl, or cycloheptyl, cyclooctyl, cyclononyl or cyclodecyl, and also as bicyclic rings or tricyclic rings, such as, for example, adamantanyl. The cycloalkyl group may also contain, one or more heteroatoms, such as oxygen, sulphur and/or nitrogen, such that a heterocycloalkyl ring is formed.

As used herein, the term "halogen" is defined in each case as fluorine, chlorine, bromine or iodine, with fluorine being preferred for compounds of formula (I) and chlorine and bromine being preferred as substituent M in compounds of formula (II).

As used herein, the term "halo-C₁-C₆-alkyl" is defined as a C₁-C₆ alkyl group wherein some or all hydrogen atoms are replaced by halogen atoms, preferably replaced with fluoro atoms. Preferred is the group CF₃.

As used herein, the term "alkenyl" is defined in each case as a straight-chain or branched alkenyl group that contains 2-6, preferably 2-4 carbon atoms. For example, the following groups can be mentioned: vinyl, propen-1-yl, propen-2-yl, but-1-en-1-yl, but-1-en-2-yl, but-2-en-1-yl, but-2-en-2-yl, 2-methyl-prop-2-en-1-yl, 2-methyl-prop-1-en-1-yl, but-1-en-3-yl, but-3-en-1-yl, and allyl.

As used herein, the term "aryl" is defined in each case has 6-12 carbon atoms, such as, for example, cyclopropenyl, cyclopentadienyl, phenyl, tropyl, cyclooctadienyl, indenyl, naphthyl, azulenyl, biphenyl, fluorenyl, anthracenyl etc, phenyl being preferred.

As used herein, the term "C₁-C₁₂", as used throughout this text e.g. in the context of the definitions of "C₁-C₁₂-alkyl" and "C₁-C₁₂-alkoxy", is to be understood as meaning an alkyl or alkoxy group having a finite number of carbon atoms of 1 to 12, i.e. 1, 2, 3, 4, 5, 6, 7, 8, 9, 10, 11 or 12 carbon atoms. It is to be understood further that said term "C₁-C₁₂" is to be interpreted as any subrange comprised therein, e.g. C₁-C₁₂ , C₂-C₁₁ , C₃-C₁₀ , C₄-C₉, C₅-C₈, C₆-C₇, C₁-C₂, C₁-C₃, C₁-C₄, C₁-C₅, C₁-C₆, C₁-C₇, C₁-C₈, C₁-C₉, C₁-C₁₀, C₁-C_{11 ;} preferably C₁-C₂ , C₁-C₃, C₁-C₄, C₁-C₅ , C₁-C_{6 ;} more preferably C₁-C₃ .

Similarly, as used herein, the term "C₂-C₆", as used throughout this text e.g. in the context of the definitions of "C₂-C₆-alkenyl", is to be understood as meaning an alkenyl group having a finite number of carbon atoms of 2 to 6, i.e. 2, 3, 4, 5, or 6 carbon atoms. It is to be understood further that said term "C₂-C₆" is to be interpreted as any subrange comprised therein, e.g. C₂-C₆ , C₃-C₅, C₃-C₄, C₂-C₃, C₂-C₄, C₂-C_{5 ;} preferably C₂-C₃.

Further as used herein, the term "C₁-C₆", as used throughout this text e.g. in the context of the definitions of "halo-C₁-C₆-alkyl" , is to be understood as meaning a haloalkyl group having a finite number of carbon atoms of 1 to 6, i.e. 1, 2, 3, 4, 5, or 6 carbon atoms. It is to be understood further that said term "C₁-C₆" is to be interpreted as any subrange comprised therein, e.g. C₁-C₆ , C₂-C₅ , C₃-C₄ , C₁-C₂, C₁-C₃, C₁-C₄ , C₁-C₅ , C₁-C_{6 ;} more preferably C₁-C₃ .

As used herein, the term "heteroaryl" as defined in each case, is an aromatic ring system which contains, in the ring, at least one heteroatom which may be identical or different, and which comprises 3-16 ring atoms, preferably 5 or 6 atoms, more preferably 9 or 10 ring atoms, said heteroatom being such as oxygen, nitrogen or sulphur, and can be monocyclic, bicyclic, or tricyclic, and in addition in each case can be benzocondensed. Preferably heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, etc., and benzo derivatives thereof, such as, e.g., benzofuranyl, benzothienyl, benzoxazolyl, benzimidazolyl, benzotriazolyl, indazolyl, indolyl, isoindolyl, etc. ; or pyridyl, pyridazinyl, pyrimidinyl, pyrazinyl, triazinyl, etc., and benzo derivatives thereof, such as, e.g., quinolinyl, isoquinolinyl, etc. ; or azocinyl, indolizinyl, purinyl, etc., and benzo derivatives thereof ; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, etc. More preferably the heteroaryl is selected from indazolyl, benzimidazolyl, quinolinyl, isoquinolinyl, benzotriazolyl. Particularly preferably, the heteroaryl is indazolyl.

The aryl group and the heteroaryl group in each case can be substituted in the same way or differently in one or more positions with halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, - SOR⁴, -COR⁶, -CO₂R⁶ or -NR⁷R⁸, whereby C₁-C₁₂-alkyl may be substituted with -OR⁵ or -NR⁷R⁸. It is understood that the substitution on the aryl group and the heteroaryl group may take place on any one of the group's carbon atoms and/or on any one of the heteroatoms. Preferably, the aryl group and the heteroaryl group is substituted in one or two positions.

If an acid group is included, the physiologically compatible salts of organic and inorganic bases are suitable as salts, such as, for example, the readily soluble alkali salts and alkaline-earth salts as well as N-methyl-glucamine, dimethyl-glucamine, ethyl-glucamine, lysine, 1,6-hexadiamine, ethanolamine, glucosamine, sarcosine, serinol, tris-hydroxy-methyl-amino-methane, aminopropanediol, Sovak base, and 1-amino-2,3,4-butanetriol.

If a basic group is included, the physiologically compatible salts of organic and inorganic acids are suitable, such as hydrochloric acid, sulphuric acid, phosphoric acid, citric acid, tartaric acid, succinic acid, fumaric acid, etc.

The compounds of general formula I according to the invention also contain the possible tautomeric forms and comprise the E-isomers or Z-isomers, (particularly it is possible that the imine II of the invention can exists as E- or Z- isomers, or as amixture thereof in any ratio), if one or more stereogenic centers are present, racemates and/or enantiomers and/or diastereoisomers. Thus, a molecule with a single stereogenic center may be a mixture of enantiomers (*R*,*S*), or may be a single (*R*) or (*S*) enantiomer. A molecule with more than one stereogenic centre may be a mixture of diastereoisomers, or may be a single diastereoisomer, whereby the diastereoisomers may also exist as mixtures of enantiomers or single enantiomers.

Within the context of the present invention the term "reducing agent" is understood as meaning a compound which is capable converting a -C=N- bond into a -CH-NH- moiety, particularly by addition of hydrogen. Particularly, said reducing agent is a hydride ion donor, such as a metal hydride, or a non-metal hydride, such as sodium hydride, sodium borohydride, sodium cyanoborohydride, sodium triacetoxyborohydride, lithium borohydride, an aluminium hydride such as super hydride, lithium dimethoxyaluminiumhydride, lithium aluminium hydride, an alkylsilane, particularly a trialkylsilane, such as triethylsilane, for example.

Such reducing agents are well known to the person skilled in the art., for example the textbook : Comprehensive Organic Synthesis, Ed. B. Trost, Pergamon Press, 1992, Vol. 8., pp. 1-183, which is hereby incorporated by reference.

Within the context of the present invention the term "acid", as optionally present in any reaction step of the method of the invention, such as the reduction of compounds with a reducing agent, e.g. in the reduction of coumpounds of formula II to give compounds of formula I, or as optionally present in the reaction of compounds of formula III with compounds of formula IV, for example, is understood as meaning any mineral acid well known to the person skilled in the art, such as, for example, hydrochloric acid, sulphuric acid, phosphoric acid, etc., or any organic acid well known to the person skilled in the art, such as, for example, a mono-, di- or tri- or poly-carboxylic acid, such as formic, acetic, propionic, butyric, pentanoic, hexanoic, heptanoic, octanoic acid, citric acid, tartaric acid, succinic acid, fumaric acid, etc., or isomers thereof, or a halogenated carboxylic acid, such as a fluorinated, chlorinated, brominated or iodinated carboxylic acid, such as trifluoroacetic acid, pentafluopriopionic acid, for example. Within the context of the present invention the term "base", as optionally present in any reaction step of the method of the invention, such as the reduction of compounds with a reducing agent, e.g. in the reduction of coumpounds of formula II to give compounds of formula I, or as optionally present in the reaction of compounds of formula III with compounds of formula IV, for example, is understood as meaning any inorganic base well known to the person skilled in the art, such as, for example, sodium carbonate, sodium bicarbonate, potassium carbonate, potassium bicarbonate, caesium carbonate, potassium phosphate, sodium tert-butoxide, etc. or any organic base well known to the person skilled in the art, such as, for example, the readily soluble alkali salts and alkaline-earth salts as well as N-methyl-glucamine, dimethyl-glucamine, ethyl-glucamine, lysine, 1,6-hexadiamine, ethanolamine, glucosamine, sarcosine, serinol, tris-hydroxy-methyl-amino-methane, aminopropanediol, Sovak base, and 1-amino-2,3,4-butanetriol.

Within the context of the present invention the term "solvent", as optionally present in any reaction step of the method of the invention, is understood, as is by the person skilled in the art, as meaning any substance in which other materials dissolve to form a solution, such as, without being limited to : a polar solvent, such as a polar protic solvent, such as water, acetic acid, n-butanol, isopropanol, n-propanol, ethanol, methanol, or formic acid or acetic acid, etc., for example ; a polar aprotic solvent, such as 1,4-dioxane, tetrahydrofuran, acetone, acetonitrile, dimethylformamide, or dimethylsulphoxide, etc., for example ; or a non-polar solvents, such as pentane, hexane, benzene, toluene, diethyl ether, methyl ethyl ketone, dichoromethane, chloroform, tetrachloromethane, ethyl acetate, etc., for example ; or any mixture of the solvents listed above.

It is understood that any combination of the definitions given in the above-mentioned embodiments is possible within the context of the present invention.

The invention will be better understood upon reading the Examples below, which are provided as an illustration of the present invention. The Examples below in no way whatsoever constitute a limitation of the present invention as described in the present text and as defined in the claims appended hereto.

### EXAMPLES

### Abbreviations used :

The following abbreviations used in the Examples have the following meanings : 1 H-NMR proton nuclear magnetic resonance spectroscopy : chemical shifts (δ) are given in ppm.
- s: singlet
- bs: broad singlet
- conc.: concentrated
- d: doublet
- dd: doublet of doublets
- h: hour / hours
- MTBE: methyl-tert-butylether
- t: triplet
- THF: tetrahydrofuran
- Xantphos: 9,9-dimethyl-4,5-bis(diphenylphosphino)xanthene

### Example 1 : Preparation of 2-chloropyridine-4-carbaldehyde dimethylacetal :

A solution of 30 g 2-chloropyridin-4-carbaldehyde (Tetrahedron Lett. 42, (2001), pp. 6815-18) in 60 ml toluene and 150 ml methanol was treated with 2 eq. trimethylorthoformate and 0.02 eq. conc. sulphuric acid and stirred at 50°C for 2 h. The reaction volume was reduced by vacuum distillation to a total volume of about 45 ml. After cooling down to room temperature the resulting oil was diluted with 100 ml ethyl acetate and washed with 50 ml aqueous sodium bicarbonate ( 10% solution). After re-extraction of the water solution with 50 ml ethyl acetate, the combined organic solutions were diluted with 150 ml toluene and distilled in vacuum to yield 39.4 g (99%) of the title compound as a colourless oil.
¹H-NMR (300 MHz, CDCl₃): δ = 3.31 (s, 6H); 5.36 (s, 1 H); 7.29 (dd, 1 H), 7.41 (d, 1 H), 8.38 (d, 1 H).

### Example 2 : Preparation of 2-Amino-4-carbaldehyde dimethylacetal :

In a pressure vessel, a slurry of 30 g 2-chloropyridine-4-carbaldehyde dimethylacetal, 300 ml ethylene glycol and 3.23 g copper (II) chloride were treated with 144 g ammonia and stirred at 120°C for 38 h. The reaction mixture was distilled with toluene at a Dean Stark trap until no glycol was separated any more. The solution was washed with 150 ml brine and after separation distilled to a volume of 45 ml. It was diluted with di-iso-propyl ether and treated with carefully with hexane. The title compound precipitated as a colourless material to yield 10.42 g (39%).
¹H-NMR (300 MHz, CDCl₃) : δ = 3.31 (s, 6H); 4.5 (bs, 2H); 5.27 (s, 1H); 6.60 (d, 1 H), 6.71 (dd, 1 H), 8.05 (d, 1 H).

### Example 3: Preparation of 3-(4-Dimethoxymethyl-pyridin-2-yl)-1,1-dimethyl-urea and 3-(4-Dimethoxymethyl-pyridin-2-yl)-1,1-dimethyl-urea :

To a slurry of 71.3 g sodium hydride (60% in mineral oil) in 600 ml THF at 55°C was added slowly a solution of 100 g 2-Amino-4-carbaldehyde dimethylacetal in 300 ml THF. After further 60 min. at this temperature it was cooled to room temperature and a solution of 83.1 g N,N-dimethylcarbamoyl chloride in 100 ml THF was added slowly. After stirring for 2 h, 300 ml of aqueous ammonia (25%) were added slowly and it was stirred for 45 min. The volume was further diluted with 150 ml water and the phases were separated. After re-extraction of the water phase with 150 ml THF the resulting organic phase was distilled in vacuum to 200 ml, 600 ml toluene were added and the solution was again distilled in vacuum to a final volume of 750 ml. The solution contains the desired 3-(4-Dimethoxymethyl-pyridin-2-yl)-1,1-dimethyl-urea and is used in the following step without further manipulation.
The toluene solution from the step before was treated with 280 ml aqueous hydrochloric acid (7%) and stirred for 15 min. The phases were separated and the toluene phase was again stirred with 150 ml aqueous hydrochloric acid (7%). The combined acidic phases were stirred for 2 hours at 85°C. After cooling down a solution of potassium phosphate in 170 ml water was added and the reaction mixture was extracted 3 times with 150 ml ethyl acetate. The combined organic phases were distilled in vacuum to 200 ml volume, 250 ml toluene was added and it was distilled to a volume of 225 ml. After adding a second charge of 250 ml toluene it was distilled again to a final volume of 220 ml. During this process, 3-(4-Dimethoxymethyl-pyridin-2-yl)-1,1-dimethyl-urea crystallised as beige material that was collected by filtration to yield 87.8 g (76.5%).
¹H-NMR (300 MHz, CDCl₃) : δ = 3.14 (s, 6H); 7.37 (s, 1 H); 7.41 (dd, 1 H); 8.42 (d, 1H), 8.59 (d, 1H), 10.08 (s, 1H).

### Example 4: Preparation of 2-Amino-N-(2-methyl-2H-indazol-6-yl)-benzamide :

A solution of 34.9 g isatinic anhydride, 30 g 6-amino-2-methyl-2H-indazole and 12.2 ml acetic acid in 94 ml 2-butanol were stirred for 6 hours at 100°C. After evaporation of 70 ml 2-butanol, 45 ml methanol were added and after further 45 min. at 65°C the reaction was cooled to room temperature. The suspension was filtered and the solution was distilled to remove the methanol. At 45 °C about 45 ml ethyl acetate and 30 ml triethylamine were added. To this solution, 250 ml MTBE were added slowly at 45°C to precipitate the title compound as a pale material that was filtered off. After drying, 47g (86%) of 2-Amino-N-(2-methyl-2H-indazol-6-yl)-benzamide were isolated.
¹H-NMR (300 MHz, CDCl₃) : δ = 4.18 (s, 3H); 6.29 (s, 2H); 6.56 (t, 1H); 6.72 (d, 1H); 7.16 (dt, 1H); 7.25 (dd, 1H); 7.55-7.65 (m, 2H); 8.05 (s, 1H); 8.20 (s, 1H); 9.95 (s, 1 H).

### Example 5 : Preparation of 2-{[1-[2-(3,3-Dimethyl-ureido)-pyridin-4-yl]-methylidene]-amino}-N-(2-methyl-2H-indazol-6-yl)- benzamide :

A solution of 10 g 2-Amino-N-(2-methyl-2H-indazol-6-yl)-benzamide, 7.26 g 3-(4-Dimethoxymethyl-pyridin-2-yl)-1,1-dimethyl-urea and 0.21 ml acetic acid in 100 ml ethanol was stirred for 24 hours at room temperature. The resulting suspension was filtered of an the resulting crude solid product was washed with 100 ml ethanol and 100 ml MTBE to yield 16.33 g (98.5%) of the title compound.
¹H-NMR (300 MHz, DMSO-d₆) : δ = 2.92 (s, 6H); 4.08 (s, 3H); 7.12 (d, 1H); 7.34 (d, 1 H); 7.38-7.46 (m, 2H); 7.54 (d, 1 H); 7.58 (t, 1 H); 7.88 (d, 1 H); 8.16 (s, 1 H); 8.20 (s, 1 H); 8.31-8.36 (m, 2H); 8.64 (s, 1 H); 9.07 (s, 1 H); 10.56 (s, 1 H).

### Example 6: 2-{[2-(3,3-Dimethyl-ureido)-pyridin-4-ylmethyl]-amino}-N-(2-methyl-2H-indazol-6-yl)- benzamide :

A solution of 5.9 g 2-{[1-[2-(3,3-Dimethyl-ureido)-pyhdin-4-yl]-methylidene]-amino}-N-(2-methyl-2H-indazol-6-yl)- benzamide in 59 ml of dichloromethane and 23.6 ml trifluoroacetic acid were treated with 2.34 ml triethylsilane at room temperature and the reaction temperature was raised to 55°C. After 24 hours, 23.6 ml of ice cold water were added and 16.2 ml sodium hydroxide solution (50%) were added. After phase separation, the water phase was extracted with dichloromethane and the combined organic solution was washed with water. The organic solution was distilled to a final volume of 35 ml and 60 ml of MTBE were added slowly. The title compound was isolated by filtration as a crystalline solid coming out of this procedure to yield 4.84 g (81.6%).
¹H-NMR (300 MHz, DMSO-d₆) : δ = 2.88 (s, 6H); 4.09 (s, 3H); 4.40 (d, 2H); 6.50 (d, 1 H); 6.62 (t, 1 H); 6.90 (d, 1 H); 7.21 (t, 1 H); 7.27 (dd, 1 H); 7.59 (d, 1 H); 7.69 (dd, 1 H); 7.79 (s, 1 H); 7.92 (t, 1 H); 8.07 (s, 1 H); 8.10 (d, 1 H); 8.22 (s, 1H); 8.75 (s, 1H); 10.11 (s, 1H).

## Claims

1. A method of preparing an anthranilamide of formula I : 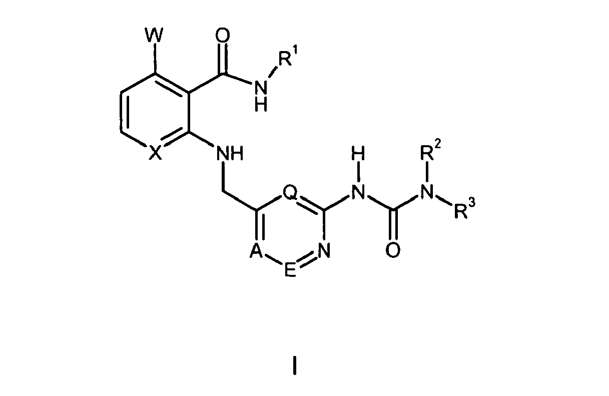 in which :
X is CH or N ;
W is hydrogen or fluorine ;
A, E and Q, independently of one another, are CH or N, whereby only a maximum of two nitrogen atoms are contained in the ring ;
R¹ is aryl or heteroaryl, which may be optionally substituted in one or more places in the same way or differently with halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴,-COR⁶, -CO₂R⁶ or -NR⁷R⁸, whereby C₁-C₁₂-alkyl may be substituted with -OR⁵ or-NR⁷R⁸,
R² and R³, independently of one another, are C₁-C₁₂ alkyl optionally substituted with -OR⁵ ;
R⁴ is C₁-C₁₂-alkyl, C₃-C₈-cycloalkyl, aryl or heteroaryl ;
R⁵ is hydrogen, C₁-C₁₂-alkyl, C₃-C₈-cycloalkyl or halo-C₁-C₆-alkyl ;
R⁶ is hydrogen, C₁-C₁₂-alkyl, C₃-C₈-cycloalkyl, halo-C₁-C₆-alkyl, aryl, or-NR⁷R⁸ ;
R⁷ and R⁸ independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, preferably a 4-7 membered cycloalkyl ring, more preferably a 5 or 6 membered cycloalkyl ring, which may optionally contain further heteroatoms, and may be optionally substituted in one or more places in the same way or differently with halogen, cyano, C₁-C₁₂-alkyl, C₁-C₁₂-alkoxy, halo-C₁-C₆-alkyl, =O, -OR⁵ , COR⁶, -SR⁴, -SOR⁴ or -SO₂R⁶ ; and
as well as isomers, diastereoisomers, enantiomers, tautomers thereof,
**characterised in that** an imine of formula II : 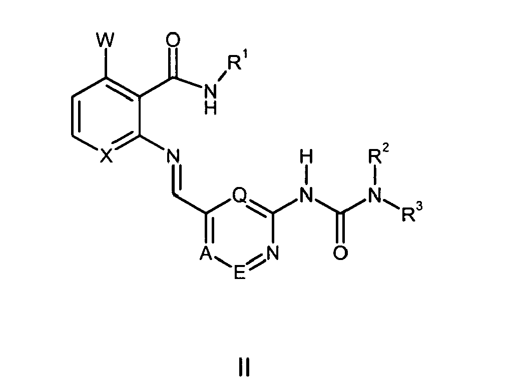 in which R¹ , R² , R³ , A, E, Q, X, and W are defined as for formula I,
is allowed to react with a reducing agent, optionally in the presence of a solvent, thereby providing an anthranilamide of formula I.

2. The method according to claim 1, wherein in said compound of formula I,
X is CH or N ;
W is hydrogen or fluorine ;
A, E and Q, independently of one another, are CH or N, whereby only a maximum of two nitrogen atoms are contained in the ring ;
R¹ is aryl or heteroaryl, which may be optionally substituted in one or more places in the same way or differently with halogen, hydroxy, C₁-C₁₂-alkyl, C₁-C₁₂-alkoxy, halo-C₁-C₆-alkyl, -SO₂R⁶, -OR⁵, COR⁶, -CO₂R⁶ or -NR⁷R⁸, whereby C₁-C₁₂-alkyl may be substituted with -OR⁵ or -NR⁷R⁸,
R² and R³, independently of one another, are C₁-C₁₂ alkyl optionally substituted with -OR⁵ ;
R⁴ is C₁-C₁₂-alkyl, C₃-C₈-cycloalkyl, aryl or heteroaryl ;
R⁵ is hydrogen, C₁-C₁₂-alkyl, C₃-C₈-cycloalkyl or halo-C₁-C₆-alkyl ;
R⁶ is aryl, or -NR⁷R⁸ ;
R⁷ and R⁸ independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, preferably a 4-7 membered cycloalkyl ring, more preferably a 5 or 6 membered cycloalkyl ring, which may optionally contain further heteroatoms, and may be optionally substituted in one or more places in the same way or differently with halogen, C₁-C₁₂-alkyl, C₁-C₁₂-alkoxy, halo-C₁-C₆-alkyl, -OR⁵ , COR⁶, -SR⁴, or -SO₂R⁶ ; and
as well as isomers, diastereoisomers, enantiomers, tautomers thereof.

3. The method according to claim 1 or 2, wherein in said compound of formula
I, X is CH ;
W is hydrogen ;
A, E and Q, independently of one another, are CH or N, whereby only a maximum of two nitrogen atoms are contained in the ring ;
R¹ is aryl or heteroaryl, which may be optionally substituted in one or more places in the same way or differently with C₁-C₁₂-alkyl, C₁-C₁₂-alkoxy,-SO₂R⁶, -OR⁵, COR⁶, or -NR⁷R⁸, whereby C₁-C₁₂-alkyl may be substituted with-OR⁵ or -NR⁷R⁸,
R² and R³, independently of one another, are C₁-C₁₂ alkyl optionally substituted with -OR⁵ ;
R⁵ is C₁-C₁₂-alkyl, or C₃-C₈-cycloalkyl ;
R⁶ is aryl, or -NR⁷R⁸ ;
R⁷ and R⁸ independently of one another, are hydrogen, -COR⁶, aryl, C₃-C₈-cycloalkyl, C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, preferably a 4-7 membered cycloalkyl ring, more preferably a 5 or 6 membered cycloalkyl ring, which may optionally contain further heteroatoms, and may be optionally substituted in one or more places in the same way or differently with halogen, C₁-C₁₂-alkyl, C₁-C₁₂-alkoxy, -OR⁵ , COR⁶, or -SO₂R⁶ ; and
as well as isomers, diastereoisomers, enantiomers, tautomers thereof.

4. The method according to any one of claims 1 to 3, wherein said compound of formula I is selected from the group consisting of :
2-{[2-(3,3-dimethyl-ureido)-pyridin-4-ylmethyl]-amino}-N-(2-methyl-2H-indazol-6-yl)-benzamide
2-{[2-(3,3-diethyl-ureido)-pyridin-4-ylmethyl]-amino}-N-(2-methyl-2H-indazol-6-yl)-benzamide
2-({2-[3-(2-hydroxy-ethyl)-3-methyl-ureido]-pyridin-4-ylmethyl}-amino)-N-(2-methyl-2H-indazol-6-yl)-benzamide
2-({2-[3-(2-methoxy-ethyl)-3-methyl-ureido]-pyridin-4-ylmethyl}-amino)-N-(2-methyl-2H-indazol-6-yl)-benzamide
2-{[2-(3-ethyl-3-methyl-ureido)-pyridin-4-ylmethyl]-amino}-N-(2-methyl-2H-indazol-6-yl)-benzamide
2-{[2-(3,3-dimethyl-ureido)-pyridin-4-ytmethyl]-amino}-N-(4-fluoro-2-methyl-2H-indazol-6-yl)-benzamide
2-{[2-(3,3-dimethyl-ureido)-pyridin-4-ylmethyl]-amino}-N-(7-methoxy-isoquinolin-3-yl)-benzamide
6-(2-{[2-(3,3-dimethyl-ureido)-pyridin-4-ylmethyl]-amino}-benzoylamino)-2-methyl-2H-indazole-3-carboxylic acid methyl ester
2-{[2-(3,3-dimethyl-ureido)-pyridin-4-ylmethyl]-amino}-N-(2-methyl-2H-benzotriazol-5-yl)-benzamide
2-{[2-(3,3-dimethyl-ureido)-pyridin-4-ylmethyl]-amino}-N-(1-methyl-2-oxo-1,2-dihydro-quinolin-6-yl)-benzamide
2-{[2-(3,3-dimethyl-ureido)-pyridin-4-ylmethyl]-amino}-N-(2-methyl-2H-indazol-7-yl)-benzamide
2-{[2-(3,3-dimethyl-ureido)-pyridin-4-ylmethyl]-amino}-N-(1-methyl-3a,7a-dihydro-1 H-indazol-4-yl)-benzamide
2-{[2-(3,3-dimethyl-ureido)-pyridin-4-ylmethyl]-amino}-N-(5-fluoro-2-methyl-2H-indazol-4-yl)-benzamide
2-{[2-(3,3-dimethyl-ureido)-pyridin-4-ylmethyl]-amino}-N-(6-fluoro-2-methyl-2H-indazol-7-yl)-benzamide
6-(2-{[2-(3,3-dimethyl-ureido)-pyridin-4-ylmethyl]-amino}-benzoylamino)-1-methyl-1H-indazole-3-carboxylic acid methyl ester
2-{[2-(3,3-dimethyl-ureido)-pyridin-4-ylmethyl]-amino}-N-(3-hydroxymethyl-1-methyl-1 H-indazol-6-yl)-benzamide
N-(3,6-difluoro-quinolin-2-yl)-2-{[2-(3,3-dimethyl-ureido)-pyridin-4-ylmethyl]-amino}-benzamide
2-{[2-(3,3-dimethyl-ureido)-pyridin-4-ylmethyl]-amino}-N-(3-sulfamoyl-phenyl)-benzamide
N-(2,3-dimethyl-2H-indazol-6-yl)-2-{[2-(3,3-dimethyl-ureido)-pyridin-4-ylmethyl]-amino}-benzamide
2-{[2-(3,3-dimethyl-ureido)-pyridin-4-ylmethyl]-amino}-N-(3-methoxymethyl-2-methyl-2H-indazol-6-yl)-benzamide
2-{[2-(3,3-dimethyl-ureido)-pyridin-4-ylmethyl]-amino}-N-(3-methoxymethyl-1-methyl-1H-indazol-6-yl)-benzamide
6-(2-{[2-(3,3-dimethyl-ureido)-pyridin-4-ylmethyl]-amino}-benzoylamino)-1-methyl-1H-indazole-3-carboxylic acid methylamide
2-{[2-(3,3-dimethyl-ureido)-pyridin-4-ylmethyl]-amino}-N-(6-fluoro-1-methyl-1 H-indazol-5-yl)-benzamide
2-{[2-(3,3-dimethyl-ureido)-pyridin-4-ylmethyl]-amino}-N-(6-fluoro-2-methyl-2H-indazol-5-yl)-benzamide
2-{[2-(3,3-dimethyl-ureido)-pyridin-4-ylmethyl]-amino}-N-(5-fluoro-1-methyl-1 H-indazol-4-yl)-benzamide
2-{[2-(3,3-dimethyl-ureido)-pyridin-4-ylmethyl]-amino}-N-quinolin-3-yl-benzamide
2-{[2-(3,3-dimethyl-ureido)-pyridin-4-ylmethyl]-amino}-N-(3-fluoro-6-methoxy-quinolin-2-yl)-benzamide
2-{[2-(3,3-dimethyl-ureido)-pyridin-4-ylmethyl]-amino}-N-(3-methyl-3H-benzoimidazol-5-yl)-benzamide
2-{[2-(3,3-dimethyl-ureido)-pyridin-4-ylmethyl]-amino}-N-(1-methyl-1H-benzoimidazol-5-yl)-benzamide
2-{[2-(3,3-Dimethyl-ureido)-pyridin-4-ylmethyl]-amino}-N-(3-methanesulfonylphenyl)-benzamide
2-{[2-(3,3-dimethyl-ureido)-pyridin-4-ylmethyl]-amino}-6-fluoro-N-(2-methyl-2H-indazol-6-yl)-benzamide, and
as well as isomers, diastereoisomers, enantiomers, tautomers and salts thereof.

5. The method according to any one of claims 1 to 4, wherein said reducing agent is a hydride ion donor, which is used optionally in the presence of an acid, or a base.

6. The method according to claim 5, wherein said hydride donor is selected from the group consisting of complex hydrides like sodium borohydride, sodium cyanoborohydride, sodium triacetoxyborohydride, lithium borohydride, an aluminium hydride such as super hydride, lithium dimethoxyaluminiumhydride, lithium aluminium hydride, an alkylsilane, particularly a trialkylsilane, such as triethylsilane, for example.

7. The method according to claim 5 or 6, wherein said acid is a mineral acid, or an organic acid, such as a carboxylic acid, such as trifluoroacetic acid, for example.

8. The method according to any one of claims 1 to 7, wherein said solvent is selected from the group consisting of water, an alcohol, an ether, a carboxylic ester, and an optionally halogenated hydrocarbon, particularly a chlorinated hydrocarbon, such as dichloromethane, for example.

9. The method according to any one of claims 1 to 8, wherein the molar ratio of said imine of formula II/said reducing agent is between 1 : 0.5 and 1:20, preferably between 1: 0.5 and 1:5, particularly between 1:1 and 1:2.

10. The method according to any one of claims 5 to 9, wherein the molar ratio of said imine of formula II/said acid is between 1:0.1 and 1:30, particularly between 8 : 1 and 30 :1.

11. The method according to any one of claims 1 to 10, which is carried out at a temperature of between the freezing point of the reaction mixture and the reflux temperature of the reaction mixture, preferably between 15°C and 110°C, particularly between room temperature amd 55°C.

12. The method according to any one of claims 1 to 11, wherein said imine of formula II is prepared by allowing an amine of formula III : 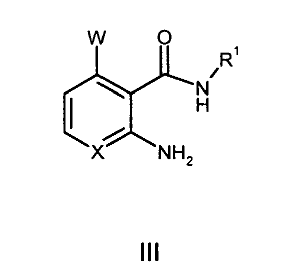 in which R¹ , X, and W are defined as for formula I in any one of claims 1 to 11,
to react, optionally in the presence of a solvent, with an aldehyde of formula IV : 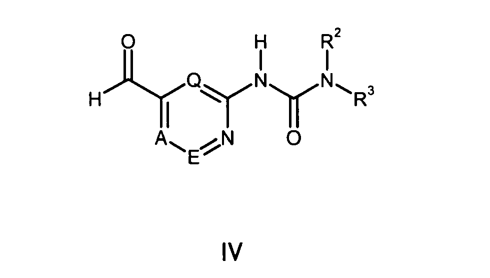 in which R² , R³ , A, E, and Q are defined as for formula I in any one of claims 1 to 11,
thereby providing an imine of formula II.

13. The method according to claim 12, which is carried out under acidic or basic conditions.

14. The method according to claim 13, wherein said acidic conditions are provided by an acid such as a carboxylic acid, such as acetic acid, for example.

15. The method according to any one of claims 12 to 14, wherein said solvent is selected from the group consisting of an alcohol, such as methanol, for example, an ether, a carboxylic ester, and an optionally halogenated hydrocarbon, particularly an alcohol, for example methanol and / or ethanol.

16. The method according to any one of claims 12 to 15, wherein the molar ratio of said amine of formula III/said aldehyde of formula IV is between 1:0.1 and 0.1:1, particularly between 1:0.7 and 0.7:1.

17. The method according to any one of claims 12 to 16, wherein said acid is present in any amount above 0 moles, preferably at a molar ratio of said amine of formula III/said acid of 1:0.0001 and 1:200, more preferably 1: 0.01 and 1:10, particularly around 1 /around 0.1.

18. The method according to any one of claims 12 to 17, which is carried out at a temperature of between the freezing point of the reaction mixture and the reflux temperature of the reaction mixture, preferably between 0°C and 100°C and particularly between 0°C and 20°C.

19. An imine of formula II : 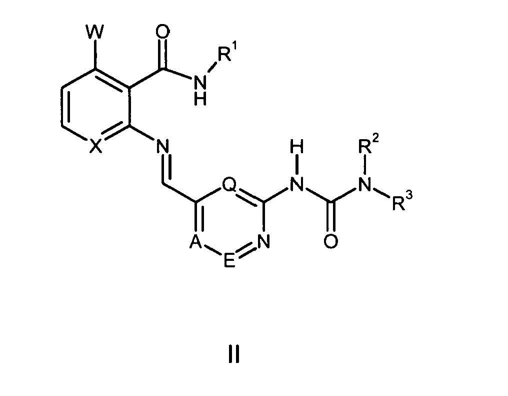 in which R¹ , R² , R³ , A, E, Q, X, and W are defined as for formula I in any one of claims 1 to 18.

20. Use of an imine according to claim 19 for the preparation of an anthranilamide of formula I according to any one of claims 1 to 18.
